Europäisches Patentamt

**European Patent Office**

**Office européen des brevets**

(19)

(11) Numéro de publication : **0 020 274**
**B1**

(12) **FASCICULE DE BREVET EUROPÉEN**

(45) Date de publication du fascicule du brevet :
22.09.82

(51) Int. Cl.³ : **A 61 K   7/13**

(21) Numéro de dépôt : **80400774.8**

(22) Date de dépôt : **30.05.80**

(54) Nouveau colorant naturel en particulier à usage capillaire et préparations cosmétiques le contenant.

(30) Priorité : 31.05.79 FR 7913970

(43) Date de publication de la demande :
10.12.80 (Bulletin 80/25)

(45) Mention de la délivrance du brevet :
22.09.82 Bulletin 82/38

(84) Etats contractants désignés :
AT BE CH DE FR GB IT LI LU NL SE

(56) Documents cités :
DE C 700 765
MELLIAND TEXTILBERICHTE, vol. 37, no. 8, août
1956 Heidelberg DE E. LUCAS : « Anwendung
verschiedener Naturfarbstoffe zum Färben von
Wolle, Zellwolle und Perlon », pages 948-950.
CHEMICAL ABSTRACTS, vol. 35, no. 7, 10 avril
1941, ref. 23319 Columbus, Ohio, US A. BUHLER :
« The manufacture of « ikat » fabrics on the
island of Rotti ».
CHEMICAL ABSTRACTS, vol. 35, no. 9, 10 mai
1941, ref. 30935 Columbus, Ohio, US
H. R. TISDALE : « Natural dyestuffs for textile
fibers ».
CHEMICAL ABSTRACTS, vol. 45, no. 17,
10 septembre 1951, ref. 77931 Columbus, Ohio,
US B. BROWN : « Ancient natural dye sources ».
CHEMICAL ABSTRACTS, vol. 82, no. 16, 21 avril
1975, page 349, colonne 2, ref 103032j Columbus,
Ohio, US.
Steinegger, Hänsel, Lehrb.d.allg.
Pharmakognosie. Berlin 1963, pp. 447-448.

(73) Titulaire : PIERRE FABRE S.A.
125, rue de la Faisanderie
F-75116 Paris (FR)

(72) Inventeur : Bellé, René
73, rue d'Aillote
F-81100 Castres (FR)

(74) Mandataire : Corre, Jacques et al
Cabinet Regimbeau 26, Avenue Kléber
F-75116 Paris (FR)

EP 0 020 274 B1

Imprimerie Jouve. 18. rue St-Denis, 75001 Paris. France

0 020 274

Colorant capillaire naturel et préparations cosmétiques le contenant

La présente invention, réalisée au Centre de Recherches PIERRE FABRE, est relative à un colorant naturel à usage capillaire, doté de propriétés additionnelles anti-inflammatoires et anti-bactériennes.

Conformément à la présente invention, le colorant capillaire est constitué par un extrait de Curcumas.

Les Curcumas, de la famille des Zingibéracées, sont des plantes vivaces, abondamment cultivées en Inde et en Chine. Leur emploi dans l'art culinaire est bien connu. En particulier, le Curcuma longa est un condiment qui entre dans la préparation du « Curry », des « pickles » etc. L'état de la technique antérieure peut en outre être illustré par les références suivantes.

Chemical Abstracts 82, 103032j (1975) décrit une composition dentifrice contenant un colorant naturel Curcumin dont la fonction n'est pas de conférer à la composition un pouvoir de coloration quelconque, mais consiste simplement à pigmenter cette composition.

La DE-AS n° 700 765 vise simplement un procédé d'extraction de drogue de Curcuma consistant en un dégraissage du végétal, reprise de l'extrait dans un alcali et précipitation par acidification de l'ensemble des pigments de Curcuma.

Lehrbuch der allgemeinen Pharmakognosie, Berlin 1963, pages 447-448 (Steinegger, Hänsel) ne vise que l'utilisation de Curcuma afin d'apporter une action contre des maladies concernant le foie et la bile et une coloration aux épices, fromages, etc.

Il apparaît ainsi qu'aucun document de technique antérieure n'avait jamais envisagé l'utilisation d'un extrait de Curcuma pour en faire un colorant capillaire naturel.

La présente invention se rapporte donc à un colorant capillaire naturel, parfaitement atoxique, et doué en outre de propriétés additionnelles anti-inflammatoires et anti-bactériennes. Ce colorant naturel consistant en un extrait de Curcuma s'est avéré dans la pratique tout à fait approprié à l'application cosmétique en tant que colorant de matières kératiniques et en particulier en tant que teinture capillaire. Il est donc susceptible d'être incorporé dans des préparations cosmétiques très diverses présentant des propriétés tinctoriales en particulier des cheveux.

La présente invention se rapporte donc également aux shampoings, lotions, sprays et autres préparations cosmétologiques incorporant le colorant précité.

Les variétés de Curcuma les plus importantes sur le plan économique et cultural, et donc particulièrement adaptées à la préparation du colorant capillaire selon l'invention, sont C. longa, C. aromatica, C. angustifolia, C. amada, C. cassia, C. domestica, C. xanthorrhiza, C. zedoaria, et C. colorata.

Conformément à la présente invention, les teintures à base de Curcuma sont de préférence obtenues, à partir des organes souterrains, par épuisement avec un solvant suffisamment polaire et volatil tel que les alcools méthylique, éthylique ou isopropylique l'acétone ou des solvants chlorés tels que le chlorure de méthylène. Après une pré-concentration du solvant et addition d'un polyol estérifié ou non, l'évaporation est continuée jusqu'à élimination complète du solvant d'extraction. Suivant le même processus, les substances actives du Curcuma appelées curcuminoïdes peuvent également être solubilisées par des huiles organiques ou minérales ou des tensio-actifs compatibles du point de vue cosmétologique.

Un dosage intermédiaire des substances actives, avant reprise par un solvant porteur, permet la dilution de l'extrait à un titre standard, convenablement choisi en fonction des effets attendus.

Selon une variante de ce procédé, les substances des Curcumas, comprenant la curcumine, la desméthoxy curcumine et la bidesméthoxy curcumine sont obtenues par extraction aqueuse en milieu basique. Après acidification et filtration, le précipité est solubilisé dans les solvants ci-dessus, de préférence dans le propanediol ou dans des tensio-actifs tels que le lauryl sulfate de sodium à 30 %.

Selon une autre variante, la nuance jaune obtenue peut être modifiée vers des nuances beige à marron, par addition de faibles quantités de sel métallique en particulier le sel de fer.

## Exemple 1

100 g de rhizomes secs finement broyés de Curcuma longa sont agités pendant 1/2 heure à reflux à deux reprises avec 500 ml de méthanol dénaturé. Après filtration les jus méthanoliques sont préconcentrés à 100 ml puis évaporés sur 250 ml environ de propanediol. L'extrait est alors clarifié, dosé en principe actif et standardisé à 0,5 % en principe colorant pour utilisation dans les spécialités cosmétiques.

## Exemple 2

100 g de poudre de Curcuma sont extraits sous agitation à 20 °C avec 700 ml de méthylisobutylcétone. Après filtration et lavage du marc par 200 ml de solvant, les filtrats groupés sont contre-extraits par 3 × 50 ml de soude après concentration. La neutralisation de la solution sodique provoque la précipitation du principe colorant, lequel est filtré, lavé à l'eau et séché. Il est ensuite introduit dans le propanediol à raison de 1 % de principe colorant.

2

## Exemple 3

100 g de poudre de Curcuma sont extraits à froid par 500 ml de soude N. Après filtration du marc, la solution sodique rouge foncée est neutralisée et le principe colorant ainsi précipité est filtré, lavé à l'eau et séché.

## Exemple 4

100 g de poudre de Curcuma sont extraits à reflux par 600 ml d'acétate d'éthyle. Le marc est lavé sur filtre par 200 ml de solvant et les phases organiques groupées sont évaporées sur 200 ml d'un ester mono-éthylique de diéthylène glycol. Après clarification celui-ci est dosé et le titre ajusté en principe actif.

On mentionnera ci-après quelques exemples de préparations cosmétologiques selon l'invention.

A titre d'exemples non limitatifs de préparations cosmétologiques selon l'invention on mentionnera ci-après deux exemples de formulation de shampooing, de baume ou de lotion.

### Exemple A (shampooing colorant)

| | | |
|---|---|---|
| Alkyl éther sulfate de sodium | 10 | % |
| Carboxy méthyl cellulose sodique | 2 | % |
| 2-bromo-2-nitro-propane-1,3-diol | 0,05 | % |
| Parfum | 0,25 | % |
| Extrait de Curcuma standardisé à 0,5 % | 2 | % |
| Eau distillée | Q.S.P. | 100 ml |

### Exemple B (shampooing colorant)

| | | |
|---|---|---|
| Alkyl éther sulfate de sodium ou de magnésium | 10 | % |
| Alkyl sorbate éthoxylé | 5 | % |
| Bentone | 0,5 | % |
| Parahydroxy benzoate | 0,15 | % |
| Parfum | 0,25 | % |
| Extrait de Curcuma standardisé à 0,5 % | 2 | % |
| Eau | Q.S.P. | 100 ml |

### Exemple C (shampooing colorant)

| | | |
|---|---|---|
| Alkyl éther sulfate de sodium | 10 | % |
| Carboxy méthyl cellulose sodique | 2 | % |
| 2-bromo-2-nitro-propane-1,3-diol | 0,05 | % |
| Parfum | 0,25 | % |
| Extrait de Curcuma standardisé à 0,5 % | 2 | % |
| Chlorure ferrique | 0,1 | % |
| Eau distillée | Q.S.P. | 100 ml |

### Exemple D (baume colorant)

Excipient :

| | | |
|---|---|---|
| Mélange d'alcools cétylique et stéarylique | 1 | % |
| Ester de glycétrol auto émulsionnable | 1,5 | % |
| Sel d'ammonium quaternaire (quaternium 7) | 2 | % |
| Propylène glycol | 1 | % |
| Alkyl polypeptide de triéthanolamine | 1 | % |
| Extrait de Curcuma standardisé à 0,5 % | 2 | % |
| Conservateurs, parfum | 0,25 | % |
| Eau distillée | Q.S.P. | 100 ml |

### Exemple E (lotion colorante à 60°)

| | | |
|---|---|---|
| Chlorure de lauryl-pyridinium | 0,045 | % |
| Octanoate de Polyglycol émulsionné | 0,10 | % |
| Résine copolymère vinylique | 0,15 | % |
| Parfum | 0,20 | % |
| Extrait de Curcuma standardisé à 0,5 % | 2 | % |
| Mélange éthanol/eau distillée 60° | Q.S.P. | 100 ml |

## Revendications

1. Colorant capillaire naturel, caractérisé en ce qu'il est extrait à partir de Curcumas.

2. Colorant capillaire selon la revendication 1, caractérisé en ce qu'il est à base de curcumine et de desméthoxy curcumine et de bidesméthoxy curcumine.

3. Colorant capillaire selon l'une des revendications 1 et 2, caractérisé en ce que lesdits Curcumas sont choisis parmi les variétés suivantes : C. longa, C. aromatica, C. angustifolia, C. amada, C. cassia, C. domestica, C. xanthorhiza, C. zedoaria, et C. colorata.

4. Colorant capillaire selon l'une des revendications 1 à 3, caractérisé en ce qu'il est obtenu par extraction des organes souterrains des Curcumas.

5. Colorant capillaire selon l'une des revendications 1 à 4, caractérisé en ce qu'il est obtenu par épuisement par un solvant suffisamment polaire et volatil, suivi d'une évaporation sur un polyol, une huile organique ou minérale, ou un tensio-actif.

6. Colorant capillaire selon l'une des revendications 1 à 4, caractérisé en ce qu'il est obtenu par extraction à l'aide d'une solution aqueuse d'un métal alcalin ou alcalino-terreux.

7. Préparation cosmétique pour la teinture des cheveux, caractérisée en ce qu'elle contient un colorant capillaire à base de curcuminoïdes selon l'une des revendications 1 à 6, éventuellement en association avec d'autres colorants naturels.

8. Préparation cosmétique selon la revendication 7, caractérisée en ce que le colorant capillaire à base de curcuminoïdes est présent sous la forme d'un extrait standardisé à 0,5 %.

9. Préparation cosmétique selon l'une des revendications 7 et 8, caractérisée en ce qu'elle contient en outre de faibles quantités de sel métallique, notamment de sel de fer.

## Claims

1. A natural capillary dye, characterised in that it is extracted from Curcumas.

2. A capillary dye according to claim 1, characterised in that it is based on curcumin and on desmethoxy curcumin and bidesmethoxy curcumin.

3. A capillary dye according to one of claims 1 and 2, characterised in that said Curcumas are selected from among the following varieties : C. longa, C. aromatica, C. angustifolia, C. amada, C. cassia, C. domestica, C. xanthorrhiza, C. zedoaria, and C. colorata.

4. A capillary dye according to one of claims 1 to 3, characterised in that it is obtained by extraction from the subterranean organs of Curcumas.

5. A capillary dye according to one of claims 1 to 4, characterised in that it is obtained by extraction using a sufficiently polar and volatile solvent, followed by evaporation on a polyol, an organic or mineral oil, or a surface-active agent.

6. A capillary dye according to one of claims 1 to 4, characterised in that it is obtained by extraction using an aqueous solution of an alkali metal or an alkaline-earth metal.

7. A cosmetic preparation for dyeing hair, characterised in that it contains a capillary dye based on curcuminoids according to one of claims 1 to 6, optionally in association with other natural dyes.

8. A cosmetic preparation according to claim 7, characterised in that the capillary dye based on curcuminoids is present in the form of an extract which is standardized to 0.5 %.

9. A cosmetic preparation according to one of claims 7 and 8, characterised in that it also contains small quantities of metallic salt, notably iron salt.

## Ansprüche

1. Natürliches Haarfärbemittel, dadurch gekennzeichnet, daß es aus Curcuma gewonnen wird.

2. Haarfärbemittel nach Anspruch 1, dadurch gekennzeichnet, daß Curcumin, Desmethoxy-Curcumin und Bidesmethoxy-Curcumin als Grundlage dienen.

3. Haarfärbemittel nach einem der Ansprüche 1 und 2, dadurch gekennzeichnet, daß die genannten Curcumen unter den folgenden Arten gewählt werden : C. longa, C. aromatica, C. angustifolia, C. amada, C. cassia, C. domestica, C. xanthorrhiza, C. zedoaria, und C. colorata.

4. Haarfärbemittel nach einem der Ansprüche 1 bis 3, dadurch gekennzeichnet, daß es durch Extraktion der unterirdischen Pflanzenteile der Curcuma erhalten wird.

5. Haarfärbemittel nach einem der Ansprüche 1 bis 4, dadurch gekennzeichnet, daß es durch Auslaugen mit einem ausreichend polaren und flüchtigen Lösungsmittel gewonnen wird, gefolgt von einer Evaporierung auf ein Polyol, ein organisches- oder ein Mineralöl oder ein oberflächenaktives Mittel.

6. Haarfärbemittel nach einem der Ansprüche 1 bis 4, dadurch gekennzeichnet, daß es durch Extraktion mit Hilfe einer wässerigen Lösung eines Alkali- oder Erdalkalimetalles hergestellt wird.

7. Kosmetisches Präparat zum Färben der Haare, dadurch gekennzeichnet, daß es ein Haarfärbemittel auf Curcumin-Basis nach einem der Ansprüche 1 bis 6 enthält, gegebenenfalls in Verbindung mit anderen natürlichen Farbstoffen.

8. Kosmetisches Präparat nach Anspruch 7, dadurch gekennzeichnet, daß das Haarfärbemittel auf Curcumin-Basis in Form eines standardisierten Extrakts zu 0,5 % vorhanden ist.

9. Kosmetisches Präparat nach einem der Ansprüche 7 und 8, dadurch gekennzeichnet, daß es außerdem geringe Mengen von Metallsalz, insbesondere von Eisensalz, enthält.